(19) **Europäisches Patentamt** / **European Patent Office** / **Office européen des brevets**

(11) **EP 4 345 832 A1**

(12) **EUROPEAN PATENT APPLICATION**

(43) Date of publication:
03.04.2024  Bulletin 2024/14

(21) Application number: 22205089.0

(22) Date of filing: 02.11.2022

(51) International Patent Classification (IPC):
*G16H 20/70* (2018.01)    *G16H 50/20* (2018.01)

(52) Cooperative Patent Classification (CPC):
G16H 50/20; G16H 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  30.09.2022  US 202263412094 P

(71) Applicant: **Koninklijke Philips N.V.
5656 AG Eindhoven (NL)**

(72) Inventors:
• **KRON, Sara E.**
**Eindhoven (NL)**
• **MOKASHI, Yash Parag**
**Eindhoven (NL)**
• **SHELLY, Benjamin Irwin**
**5656AG Eindhoven (NL)**

(74) Representative: **Philips Intellectual Property &
Standards
High Tech Campus 52
5656 AG Eindhoven (NL)**

(54)   **SYSTEM AND METHOD FOR PROVIDING SLEEP CONSOLIDATION THERAPY**

(57)   A method of administering sleep consolidation therapy to a patient to treat a sleep disorder of the patient. The method includes monitoring, via a first number of sensors, a number of characteristics and/or activities of the patient during a number of wake periods of the patient. The method further includes determining, at least in-part from the monitoring of the number of wake periods, a recommended bedtime for the patient for starting a particular sleep period and providing the recommended bedtime for starting the particular sleep period to the patient.

FIG. 1

EP 4 345 832 A1

**Description**

FIELD OF THE INVENTION

The present invention relates to systems and methods for treating conditions, such as insomnia, using sleep consolidation therapy, and more particularly, to systems and methods for dynamically providing sleep consolidation therapy.

DESCRIPTION OF THE RELATED ART

Sleep consolidation (or sleep restriction) therapy is a behavioral treatment for insomnia that works to decrease variability in the timing of sleep while increasing the depth of sleep. The goal of sleep consolidation therapy is to shorten the amount of time spent by a patient in bed in order to consolidate sleep. In traditional sleep consolidation therapy, an individual determines the allowed time in bed (TIB) by keeping a sleep log for two weeks and calculating the average total sleep time (TST). A bedtime is set by counting back from the desired wake time, which itself must be strictly adhered to, by the amount of TIB allowed (average TST + 30 minutes). Patients must stick to this sleep schedule as closely as possible for at least two weeks before assessing sleep efficiency (SE) to determine if a 15-min adjustment should be made to the TIB. The aim of sleep consolidation therapy is to build up sleep pressure by using sleep restriction.

Sleep consolidation therapy is indicated for patients having sleep difficulties in which the subjective sleep efficiency based on a 1- to 2-week sleep log or retrospective report is less than 85% (or less than 80% in older individuals). One of the most reliable ways to strengthen the homeostatic sleep drive and thereby increase the propensity for sleep during upcoming nights is to limit the amount of sleep currently being accumulated. Restricting time in bed over a number of nights is a simple way to limit sleep accumulation. Sleep restriction also redresses such indicators of poor sleep as elevated amounts of light stage N1 sleep, prolonged sleep latencies, and excessive wakefulness after sleep onset. Rapid sleep onset and a well-consolidated night of quality sleep, core goals of insomnia treatment, are achieved rapidly and reliably at the start of sleep consolidation therapy. According to the 3P model of insomnia, behavioral practices and cognitive tendencies that perpetuate sleep disturbance are often the most promising targets for intervention. Many of these perpetuating factors, such as spending too much time in bed, anticipatory anxiety about the prospects for sleep, and inordinate concern about daytime performance deficits, are addressed by sleep consolidation therapy. As noted above, sleep consolidation therapy quickly changes the experience of insomnia, replacing sleep that has been haphazard and light with deeper, more consolidated sleep.

Traditional sleep consolidation therapy recommendations are driven by the previous week's sleep efficiency and can be difficult for a patient to follow due to the stringent and rigid bed and wake times prescribed. While this therapy is the most effective sleep hygiene technique available for treating insomnia, it takes several weeks of diligent dedication by a patient to altering their own sleep schedule before positive outcomes are observed. Patients may experience increased daytime sleepiness and disrupted sleep initially, resulting in decreased job performance, daytime functioning, etc. These effects can be too difficult for some patients to reconcile, such that sleep consolidation therapy is discontinued before it can be beneficial. Hence, there is room for improvement in therapies for treating sleep disorders, such as sleep consolidation therapy.

SUMMARY OF THE INVENTION

Embodiments of the present invention provide a method of administering sleep consolidation therapy to a patient to treat a sleep disorder of the patient. The method comprises: monitoring, via a first number of sensors, a number of characteristics and/or activities of the patient during a number of wake periods of the patient; determining, at least in-part from the monitoring of the number of wake periods, a recommended bedtime for the patient for starting a particular sleep period; and providing the recommended bedtime for starting the particular sleep period to the patient.

The method may further comprise monitoring, via a second number of sensors, a number of characteristics and/or activities of the patient during a number of sleep periods, each sleep period occurring immediately following a corresponding prior wake period of the number of wake periods, wherein the recommended bed time for the patient for starting the particular sleep period is determined from the monitoring of at least a wake period of the number of wake periods and a sleep period of the number of sleep periods.

The number of wake periods may comprise a plurality of wake periods; the number of sleep periods may comprise a plurality of sleep periods; and the recommended bedtime for the patient for starting the particular sleep period may be determined from the monitoring of more than one wake period of the plurality of wake periods and more than one sleep period of the number of sleep periods.

The number of wake periods may comprise a plurality of wake periods; the number of sleep periods may comprise a plurality of sleep periods; and the recommended bedtime for the patient for starting the particular sleep period may be determined from the monitoring of at least one wake period of the plurality of wake periods and at least one sleep period of the number of sleep periods.

Monitoring the number of characteristics and/or activities of the patient during the number of wake periods may comprise monitoring the number of characteristics of the user.

Monitoring the number of characteristics and/or activities of the patient during the number of wake periods may comprise monitoring the number of activities of the user.

The first number of sensors may be positioned in a wearable device positioned on the patient.

The first number of sensors and the second number of sensors may be positioned in a wearable device positioned on the patient.

The wearable device may comprise a smartwatch.

Embodiments of the present invention also provide a system structured to administer a sleep consolidation therapy to a patient to treat a sleep disorder of the patient. The system comprises: a processor apparatus comprising a processor and a memory; an input apparatus structured to provide input signals to the processor apparatus; and an output apparatus structured to receive output signals from the processor apparatus, wherein the memory has stored therein instructions which, when executed on the processor, cause the machine to perform operations comprising: monitoring, from signals received via the input apparatus from a first number of sensors, a number of characteristics and/or activities of the patient during a number of wake periods of the patient; determining, at least in-part from the monitoring of the number of wake periods, a recommended bed time for the patient for starting a particular sleep period of the patient; and providing the recommended bed time for starting the particular sleep period to the patient via the output apparatus.

The instructions, when executed on the processor, may cause the machine to perform further operations comprising monitoring, from signals received via the input apparatus form a second number of sensors, a number of characteristics and/or activities of the patient during a number of sleep periods of the patient, each sleep period occurring immediately following a corresponding prior wake period of the number of wake periods of the patient, wherein the recommended bed time for the patient for starting the particular sleep period is determined from the monitoring of at least a wake period of the number of wake periods and a sleep period of the number of sleep periods.

The number of wake periods may comprise a plurality of wake periods; the number of sleep periods may comprise a plurality of sleep periods; and the recommended bedtime for the patient for starting the particular sleep period may be determined from the monitoring of more than one wake period of the plurality of wake periods and more than one sleep period of the number of sleep periods.

The number of wake periods may comprise a plurality of wake periods; the number of sleep periods may comprise a plurality of sleep periods; and the recommended bedtime for the patient for starting the particular sleep period may be determined from the monitoring of at least one wake period of the plurality of wake periods and at least one sleep period of the number of sleep periods.

Embodiments of the present invention further provide a non-transitory machine-readable storage medium having stored thereon instructions which, when executed on a processor apparatus of a machine that is structured to administer a sleep consolidation therapy to a patient to treat a sleep disorder of the patient, cause the machine to perform operations comprising: monitoring, via a first number of sensors, a number of characteristics and/or activities of the patient during a number of wake periods of the patient; determining, at least in-part from the monitoring of the number of wake periods, a recommended bed time for the patient for starting a particular sleep period of the patient; and providing the recommended bed time for starting the particular sleep period to the patient.

The instructions, when executed on the processor, may cause the machine to perform further operations comprising monitoring, from signals received via the input apparatus from a second number of sensors, a number of characteristics and/or activities of the patient during a number of sleep periods of the patient, each sleep period occurring immediately following a corresponding prior wake period of the number of wake periods of the patient, wherein the recommended bed time for the patient for starting the particular sleep period is determined from the monitoring of at least a wake period of the number of wake periods and a sleep period of the number of sleep periods.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various FIGS. It is to be expressly understood, however, that the drawings are for the purpose of illustration and description only and are not intended as a definition of the limits of the invention.

BRIEF DESCRIPTION OF THE DRAWINGS

FIG. 1 is schematic depiction of an improved system in accordance with an example embodiment of the present invention;

FIG. 2 is a flowchart depicting certain aspects of an improved method in accordance with an example embodiment the present invention; and

FIG. 3 is a flowchart showing an arrangement of modules in accordance with one example embodiment of the present invention.

DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

As used herein the term "wake period" refers to a period of time in which a patient is awake, while the term "sleep period" refers to a period of time in which a patient is, or is attempting to be, sleeping. For example, a patient who goes to bed at 11 PM and has a wake up time at 7 AM would be said to have a wake period from 7 AM to 11 PM and a corresponding sleep period from 11 PM to 7 AM.

As discussed in the Background, sleep consolidation (or sleep restriction) therapy has been shown to be associated with reduced objective total sleep time, increased daytime sleepiness, and objective performance impairment. In traditional sleep consolidation therapy, as the titration proceeds, a point of maximal net benefit is eventually reached over an extended period (e.g., weeks/months) of time. Embodiments of the present invention optimize sleep consolidation therapy to increase safety and ease of, and increase adherence to, the therapy by using accumulated sleep debt and predicted sleep quality to produce recommendations in order to optimize the net benefit of sleep consolidation throughout the duration of therapy. Sleep quality predictions and recommended sleep times are developed by monitoring details of the patient's waking hours (i.e., wake periods) and corresponding sleep overtime (i.e., sleep periods) through various potential inputs.

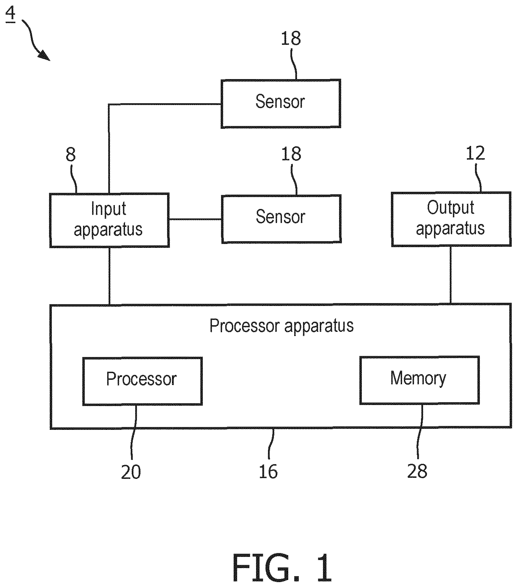

A system 4 in accordance with one example embodiment of the present invention is depicted generally in FIG. 1. System 4 can be said to be operable to administer sleep consolidation therapy to a patient to treat a sleep disorder of the patient by dynamically providing a directed sleep time to the patient for a particular sleep period.

Continuing to refer to FIG. 1, system 4 includes an input apparatus 8, an output apparatus 12, and a processor apparatus 16. Input apparatus 8 is in communication with processor apparatus 16 and can be any of a wide variety of input devices that might include, for example and without limitation, keyboards (physical and/or touchscreen), CD ROM readers, electronic data interfaces that follow any of a variety of wired or wireless protocols (e.g., without limitation, Small Computer Systems Interface (SCSI), IEEE 802.11, Ethernet, or any other suitable arrangement), as well as any other suitable input device(s), all of which are configured to provide input signals to processor apparatus 16. As shown in the example embodiment shown in FIG. 1, system 4 may include a number of sensors 18 in wired or wireless communication with processor apparatus 16 (either directly or via input apparatus 8) for actively or passively monitoring characteristics, activity, or any other suitable data of a patient. Some examples of such sensors 18 are discussed below.

Output apparatus 12 is in communication with processor apparatus 16 and can be any of a wide variety of devices and may include, for example, video displays, printers, data interfaces such as those set forth in the preceding sentence, and other output devices that all receive output signals from processor apparatus 16. Output apparatus 12 and input apparatus 8 may be provided as a single arrangement/unit that functions as both an input apparatus 8 and an apparatus 12. For example, without limitation, a smartwatch may be used to receive input from a patient, monitor various metrics of a patient, and provide output to a patient.

In the example embodiment shown in FIG. 1, processor apparatus 16 includes a processor 20 and a memory 24 that are in communication with one another. Processor 20 may be, for example and without limitation, a microprocessor, a microcontroller, or some other suitable processing device or circuitry, that interfaces with memory 24. Memory 24 can be any of one or more of a variety of types of internal and/or external storage media such as, without limitation, RAM, ROM, EPROM(s), EEPROM(s), FLASH, and the like that provide a storage register, i.e., a machine readable medium, for data storage such as in the fashion of an internal storage area of a computer, and can be volatile memory or nonvolatile memory. Memory 24 has stored therein a set of instructions that are generally in the form of routines or other types of instructions which, when executed on/by processor 20, cause system 4 to perform certain predetermined functions. Other variations will be apparent to one of ordinary skill in the art.

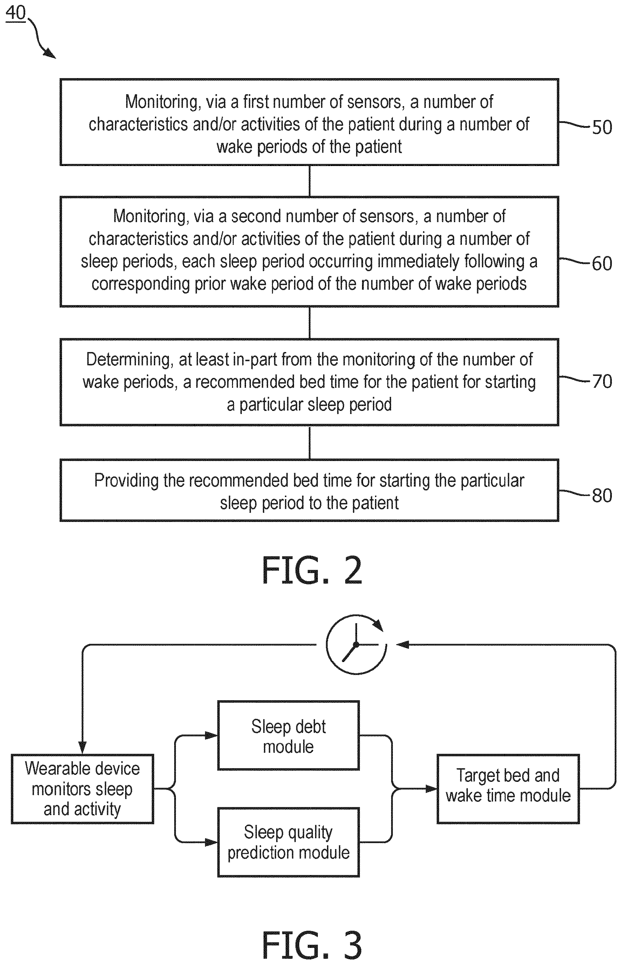

Having thus described a basic arrangement of system 4 in accordance with one example embodiment of the present invention, certain operations of system 4 will now be described below in conjunction with an exemplary flowchart of a method 40 in accordance with one example embodiment of the present invention for administering sleep consolidation therapy to a patient to treat a sleep disorder of the patient that is depicted generally in FIG. 2. As generally shown at 50, as an initial step a number of characteristics and/or activities of a patient is/are monitored during a number of wake periods of the patient via first number of sensors 18. The data obtained via such monitoring can be processed via processor 20 and stored in memory 28 as part of modules such as described further below.

As another step, as generally shown at 60, a number of characteristics and/or activities of the patient is/are monitored during a number of sleep periods of the patient, each sleep period occurring immediately following a corresponding prior wake period of the number of previously mentioned wake periods in 50, via a second number of sensors 18. Such monitoring in 50 and 60 may be carried out by one or more of the same sensors or via different sensors without varying from the scope of the present invention.

As shown at 70, a recommended bedtime for the patient for starting a particular sleep period is determined at least in-part from the monitoring of the number of wake periods of the patient in 50. The recommended bedtime can also be determined at least in-part from the monitoring of the number of sleep periods of the patient in 60. Finally, as shown at 80, the recommended bedtime for starting the particular sleep period is then provided to the patient, such as via output apparatus 12.

The example methodology discussed above and corresponding portions of system 4 can be embodied by one or more modules that may be used to carry out all or portions of example embodiments of the present invention. The following are some example of such modules in accordance with example embodiments of the present invention:

**Sleep architecture detection module** includes any biometric sensing wearable device(s) and corresponding algorithm(s) for detecting sleep architecture, including aspects of sleep architecture such as total sleep time (TST), sleep onset latency (SOL), wake after sleep onset (WASO) and sleep fragmentation;

**Activity detection module** includes any monitoring devices, which include on-body automatic detection and subjective input, and corresponding algorithms for detecting the occurrence and timing of a user's daily living activities, such as exercise, caffeine intake, alcohol intake, light exposure, timing of meals, etc.;

**Sleep debt module** calculates a user's sleep debt based on homeostatic sleep drive, circadian regulation, previous week's sleep history, and daily activities;

**Sleep quality prediction module** calculates a user's predicted sleep quality based on the previous week's sleep architecture and the current day's activities; and

**Target bed and wake time module** calculates the optimal bedtime and wake time for a user based on sleep architecture history and dynamically incorporating data from the sleep debt and sleep quality prediction modules.

A flow chart of an arrangement of some of such modules in accordance with one example embodiment of the present invention is shown schematically in FIG. 3.

In an example embodiment of the present invention, the increase of sleep consolidation therapy effectiveness for a specific insomnia patient is targeted. In such example, the effectiveness of the therapy is evaluated by monitoring and detecting changes in sleep efficiency (SE) of the patient and adherence to recommendations provided to the patient. It should be noted that sleep architecture characteristics other than SE, e.g., without limitation, SOL, can be used to detect effectiveness. Such arrangement can include/utilize:

**User interface,** such as a mobile application, which can be used by the user to provide timing and dosage of caffeine and alcohol intake, provide subjective feedback, and interact with therapy recommendations;

**Sleep tracker** for detecting sleep architecture, such as a wrist worn wearable device, under-the-mattress sensor, etc.;

**A wearable sensor for light exposure detection** providing duration, intensity and timing of both sun and artificial light detected up to the user's bedtime;

**A stress detector** providing stress level: the stress level can be provided by third party (e.g. API call from wearable) or an on-device algorithm can be deployed. In one embodiment, stress detection is assessed using heart rate variability. In another embodiment, stress detection is assessed using skin conductance. In a third embodiment, stress is subjectively self-reported by the user (e.g. by periodic questionnaire deployed on mobile application);

**An activity tracker** providing the timing and number of minutes that the user engaged in aerobic activity (e.g.,

aerobic activity being characterized by heart rate level between 55% and 85% of the user's maximum heart rate. The activity tracker may or may not have the ability to provide information about the timing of meals and substances consumed. If the activity tracker does not provide such information - **User interface module for self-reporting of food, alcohol, and caffeine intake** dosage and timing;

**Algorithm for calculating current sleep debt** utilizes data from the user's past week including the current day to determine current sleep debt and homeostatic sleep drive.

**User interface module for self-reporting desired wake time** to be used in target parameter algorithms;

**Target wake time calculation** uses the input from the user interface, if reported, to set the target wake time for the user. If a user does not report a desired wake time, the wake time can be set to a default time. Wake time should remain the same regardless of the amount of time slept but should never fluctuate by more than 1-hour past the normal wake up time (such as on days off from work or on weekends when users might want to sleep in);

**Time in bed (TIB) algorithm** uses data from the sleep debt algorithm and sleep quality prediction algorithm to determine the time a user should spend in bed to optimize sleep efficiency. Initially, TIB should be set to the sleep tracker reported average TST of the previous week. This recommendation should be updated every n-hours the user is awake to account for changes to the sleep debt and sleep quality prediction algorithms.

**Target bedtime calculation** uses variables provided throughout the system to calculate a personalized target bedtime. User-reported desired wake time and system determined TIB are used to calculate the recommended bedtime for a particular night, according to:

$$\text{Bedtime} = \text{Wake Time} - \text{TIB} \, (+ \, 24 \text{ if negative})$$

**Adherence detection algorithm** uses sleep tracked determined bedtime and wake time to calculate adherence to the user-specific recommendations for bed and wake times. If a user is consistently adhering to their recommendations, the following should be used to evaluate whether their TIB should be adjusted. This evaluation can be performed every 2-weeks;

**User interface for behavior change recommendations** incentivizes healthy behaviors by providing user behavior and activity recommendations that, when performed, shift the recommended bedtime (e.g. if you do 1-hour of aerobic activity before 12pm, your bedtime will move from 12am to 11:30pm).

As follows examples of behavioral changes recommendations are provided

Engage in an outdoor activity (e.g. walking) at least 30 mins a day
Turn-off artificial lights two hours before you go to bed
Reduce your stress by engaging in breathing exercises
Avoid caffeine intake after 1pm

The effect each of these recommendations will have depends on the specific sleep debt and sleep quality prediction curves of a user. Behavioral change recommendations are provided only for the actual behaviors that are being monitored - if the system does not include a light sensor then light exposure recommendations will not be provided, for example.

**User interface for self-reported experience** provides user feedback on bed and wake time recommendations and subjective feelings of sleep quality and is incorporated into the recommendation generating algorithms to influence therapy recommendations.

It is contemplated that aspects of the disclosed concept can be embodied as computer readable codes on a tangible computer readable recording medium. The computer readable recording medium is any data storage device that can store data which can be thereafter read by a computer system. Examples of the computer readable recording medium include, for example, without limitation, RAM, ROM, EPROM(s), EEPROM(s), FLASH, and the like that provide a storage register, i.e., a machine readable medium, for data storage such as in the fashion of an internal storage area of a computer, and can be volatile memory or nonvolatile memory.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

**Claims**

1. A method of administering sleep consolidation therapy to a patient to treat a sleep disorder of the patient, the method comprising:

   monitoring, via a first number of sensors, a number of characteristics and/or activities of the patient during a number of wake periods of the patient;
   determining, at least in-part from the monitoring of the number of wake periods, a recommended bedtime for the patient for starting a particular sleep period; and
   providing the recommended bedtime for starting the particular sleep period to the patient.

2. The method of claim 1, further comprising monitoring, via a second number of sensors, a number of characteristics and/or activities of the patient during a number of sleep periods, each sleep period occurring immediately following a corresponding prior wake period of the number of wake periods,
   wherein the recommended bedtime for the patient for starting the particular sleep period is determined from the monitoring of at least a wake period of the number of wake periods and a sleep period of the number of sleep periods.

3. The method of claim 2, wherein:

   the number of wake periods comprises a plurality of wake periods;
   the number of sleep periods comprises a plurality of sleep periods; and
   the recommended bedtime for the patient for starting the particular sleep period is determined from the monitoring of more than one wake period of the plurality of wake periods and more than one sleep period of the number of sleep periods.

4. The method of claim 2, wherein:

   the number of wake periods comprises a plurality of wake periods;
   the number of sleep periods comprises a plurality of sleep periods; and
   the recommended bedtime for the patient for starting the particular sleep period is determined from the monitoring of at least one wake period of the plurality of wake periods and at least one sleep period of the number of sleep periods.

5. The method of claim 1, wherein monitoring the number of characteristics and/or activities of the patient during the number of wake periods comprises monitoring the number of characteristics of the user.

6. The method of claim 1, wherein monitoring the number of characteristics and/or activities of the patient during the number of wake periods comprises monitoring the number of activities of the user.

7. The method of claim 1, wherein the first number of sensors are positioned in a wearable device positioned on the patient.

8. The method of claim 2, wherein the first number of sensors and the second number of sensors are positioned in a wearable device positioned on the patient.

9. The method of claim 7, wherein the wearable device comprises a smartwatch.

10. A system structured to administer a sleep consolidation therapy to a patient to treat a sleep disorder of the patient, the system comprising:

    a processor apparatus comprising a processor and a memory;

an input apparatus structured to provide input signals to the processor apparatus; and
an output apparatus structured to receive output signals from the processor apparatus,
wherein the memory has stored therein instructions which, when executed on the processor, cause the machine to perform operations comprising:

> monitoring, from signals received via the input apparatus from a first number of sensors, a number of characteristics and/or activities of the patient during a number of wake periods of the patient;
> determining, at least in-part from the monitoring of the number of wake periods, a recommended bedtime for the patient for starting a particular sleep period of the patient; and
> providing the recommended bedtime for starting the particular sleep period to the patient via the output apparatus.

11. The system of claim 10, wherein the instructions, when executed on the processor, cause the machine to perform further operations comprising monitoring, from signals received via the input apparatus from a second number of sensors, a number of characteristics and/or activities of the patient during a number of sleep periods of the patient, each sleep period occurring immediately following a corresponding prior wake period of the number of wake periods of the patient,
wherein the recommended bedtime for the patient for starting the particular sleep period is determined from the monitoring of at least a wake period of the number of wake periods and a sleep period of the number of sleep periods.

12. The system of claim 11, wherein:

> the number of wake periods comprises a plurality of wake periods;
> the number of sleep periods comprises a plurality of sleep periods; and
> the recommended bedtime for the patient for starting the particular sleep period is determined from the monitoring of more than one wake period of the plurality of wake periods and more than one sleep period of the number of sleep periods.

13. The system of claim 11, wherein:

> the number of wake periods comprises a plurality of wake periods;
> the number of sleep periods comprises a plurality of sleep periods; and
> the recommended bedtime for the patient for starting the particular sleep period is determined from the monitoring of at least one wake period of the plurality of wake periods and at least one sleep period of the number of sleep periods.

14. A non-transitory machine-readable storage medium having stored thereon instructions which, when executed on a processor apparatus of a machine that is structured to administer a sleep consolidation therapy to a patient to treat a sleep disorder of the patient, cause the machine to perform operations comprising:

> monitoring, via a first number of sensors, a number of characteristics and/or activities of the patient during a number of wake periods of the patient;
> determining, at least in-part from the monitoring of the number of wake periods, a recommended bedtime for the patient for starting a particular sleep period of the patient; and
> providing the recommended bedtime for starting the particular sleep period to the patient.

15. The non-transitory machine-readable storage medium of claim 14, wherein the instructions, when executed on the processor, cause the machine to perform further operations comprising monitoring, from signals received via the input apparatus from a second number of sensors, a number of characteristics and/or activities of the patient during a number of sleep periods of the patient, each sleep period occurring immediately following a corresponding prior wake period of the number of wake periods of the patient,
wherein the recommended bedtime for the patient for starting the particular sleep period is determined from the monitoring of at least a wake period of the number of wake periods and a sleep period of the number of sleep periods.

FIG. 1

40

| Monitoring, via a first number of sensors, a number of characteristics and/or activities of the patient during a number of wake periods of the patient | 50 |

| Monitoring, via a second number of sensors, a number of characteristics and/or activities of the patient during a number of sleep periods, each sleep period occurring immediately following a corresponding prior wake period of the number of wake periods | 60 |

| Determining, at least in-part from the monitoring of the number of wake periods, a recommended bed time for the patient for starting a particular sleep period | 70 |

| Providing the recommended bed time for starting the particular sleep period to the patient | 80 |

# FIG. 2

Wearable device monitors sleep and activity → Sleep debt module, Sleep quality prediction module → Target bed and wake time module

# FIG. 3

Europäisches Patentamt

European Patent Office

Office européen des brevets

**Application Number**

EP 22 20 5089

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/215299 A1 (MYLLYMÄKI TERO [FI] ET AL) 9 July 2020 (2020-07-09) | 1-5,7-15 | INV. G16H20/70 G16H50/20 |
| Y | * paragraphs [0052], [0053], [0089], [0092], [0097] * | 6 | |
| A | EP 3 841 967 A1 (BURTON DAVID [AU]) 30 June 2021 (2021-06-30) * the whole document * | 1-15 | |
| A | US 2022/133221 A1 (LAWLOR COLIN [US] ET AL) 5 May 2022 (2022-05-05) * the whole document * | 1-15 | |
| Y | WO 2021/061655 A1 (DELOS LIVING LLC [US]) 1 April 2021 (2021-04-01) * paragraph [0131] * | 6 | |
| A | WO 2021/234037 A1 (RESMED SENSOR TECH LTD [IE]) 25 November 2021 (2021-11-25) * the whole document * | 1-15 | |
| A | CA 3 176 857 A1 (EISAI R&D MAN CO LTD [JP]) 30 September 2021 (2021-09-30) * the whole document * | 1-15 | TECHNICAL FIELDS SEARCHED (IPC) G16H |
| A | WO 2021/220247 A1 (RESMED SENSOR TECH LTD [IE]) 4 November 2021 (2021-11-04) * the whole document * | 1-15 | |
| A | CN 108 135 534 B (RESMED SENSOR TECH LTD) 5 April 2022 (2022-04-05) * the whole document * | 1-15 | |

The present search report has been drawn up for all claims

| Place of search | Date of completion of the search | Examiner |
|---|---|---|
| Munich | 3 August 2023 | Laub, Christoph |

**ANNEX TO THE EUROPEAN SEARCH REPORT
ON EUROPEAN PATENT APPLICATION NO.**                     EP 22 20 5089

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2023

| Patent document cited in search report | | Publication date | Patent family member(s) | | Publication date |
|---|---|---|---|---|---|
| US 2020215299 | A1 | 09-07-2020 | EP | 3677171 A1 | 08-07-2020 |
| | | | US | 2020215299 A1 | 09-07-2020 |
| EP 3841967 | A1 | 30-06-2021 | AU | 2016205850 A1 | 27-07-2017 |
| | | | AU | 2018250529 B2 | 30-04-2020 |
| | | | CA | 2968645 A1 | 14-07-2016 |
| | | | CA | 3080600 A1 | 14-07-2016 |
| | | | CA | 3081166 A1 | 14-07-2016 |
| | | | CA | 3156908 A1 | 14-07-2016 |
| | | | CN | 107438398 A | 05-12-2017 |
| | | | CN | 112998649 A | 22-06-2021 |
| | | | CN | 112998650 A | 22-06-2021 |
| | | | EP | 3242587 A1 | 15-11-2017 |
| | | | EP | 3841967 A1 | 30-06-2021 |
| | | | JP | 2018505759 A | 01-03-2018 |
| | | | JP | 2021121323 A | 26-08-2021 |
| | | | JP | 2021121324 A | 26-08-2021 |
| | | | KR | 20170129689 A | 27-11-2017 |
| | | | KR | 20210008848 A | 25-01-2021 |
| | | | KR | 20220082852 A | 17-06-2022 |
| | | | WO | 2016110804 A1 | 14-07-2016 |
| US 2022133221 | A1 | 05-05-2022 | US | 2022105307 A1 | 07-04-2022 |
| | | | US | 2022108797 A1 | 07-04-2022 |
| | | | US | 2022133221 A1 | 05-05-2022 |
| | | | US | 2022139544 A1 | 05-05-2022 |
| | | | WO | 2022076670 A1 | 14-04-2022 |
| | | | WO | 2022076677 A1 | 14-04-2022 |
| | | | WO | 2022076696 A1 | 14-04-2022 |
| | | | WO | 2022076706 A1 | 14-04-2022 |
| WO 2021061655 | A1 | 01-04-2021 | US | 2022401689 A1 | 22-12-2022 |
| | | | WO | 2021061655 A1 | 01-04-2021 |
| WO 2021234037 | A1 | 25-11-2021 | EP | 4153043 A1 | 29-03-2023 |
| | | | US | 2023190140 A1 | 22-06-2023 |
| | | | WO | 2021234037 A1 | 25-11-2021 |
| CA 3176857 | A1 | 30-09-2021 | CA | 3176857 A1 | 30-09-2021 |
| | | | CN | 115668398 A | 31-01-2023 |
| | | | EP | 4128276 A1 | 08-02-2023 |
| | | | JP | 2023520335 A | 17-05-2023 |
| | | | KR | 20220159430 A | 02-12-2022 |
| | | | TW | 202141522 A | 01-11-2021 |
| | | | US | 2023148956 A1 | 18-05-2023 |
| | | | WO | 2021195316 A1 | 30-09-2021 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 20 5089

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2023

| Patent document cited in search report | Publication date | Patent family member(s) | | Publication date |
|---|---|---|---|---|
| WO 2021220247 A1 | 04-11-2021 | AU | 2021265344 A1 | 08-12-2022 |
| | | CN | 115836358 A | 21-03-2023 |
| | | EP | 4143849 A1 | 08-03-2023 |
| | | US | 2023173221 A1 | 08-06-2023 |
| | | WO | 2021220247 A1 | 04-11-2021 |
| CN 108135534 B | 05-04-2022 | CN | 108135534 A | 08-06-2018 |
| | | CN | 114588445 A | 07-06-2022 |
| | | EP | 3340876 A2 | 04-07-2018 |
| | | EP | 3838138 A2 | 23-06-2021 |
| | | JP | 6987042 B2 | 22-12-2021 |
| | | JP | 7284782 B2 | 31-05-2023 |
| | | JP | 2018531055 A | 25-10-2018 |
| | | JP | 2021180853 A | 25-11-2021 |
| | | US | 2020297955 A1 | 24-09-2020 |
| | | WO | 2017032873 A2 | 02-03-2017 |

EPO FORM P0459

For more details about this annex : see Official Journal of the European Patent Office, No. 12/82